(19) Europäisches Patentamt
European Patent Office
Office européen des brevets

(11) Veröffentlichungsnummer: **0 567 849 A1**

## (12) EUROPÄISCHE PATENTANMELDUNG

(21) Anmeldenummer: **93106056.0**

(22) Anmeldetag: **14.04.93**

(51) Int. Cl.5: **C09B 26/06**, C09B 56/20, C09B 41/00

(30) Priorität: **27.04.92 US 874674**
**15.12.92 DE 4242428**

(43) Veröffentlichungstag der Anmeldung:
**03.11.93 Patentblatt 93/44**

(84) Benannte Vertragsstaaten:
**CH DE FR GB LI**

(71) Anmelder: **BAYER AG**
**D-51368 Leverkusen(DE)**

(72) Erfinder: **Raue, Roderich, Dr.**
**Bertha-von-Suttner-Strasse 48**
**W-5090 Leverkusen(DE)**
Erfinder: **Lange, Karl-Heinrich, Dr.**
**Kastanienallee 18a**
**W-5093 Burscheid 2(DE)**
Erfinder: **Brack, Alfred, Dr.**
**Auf dem Krahwinkel 7**
**W-5068 Odenthal(DE)**

### (54) Verfahren zur Herstellung von Farbstoffen.

(57) Mit dem neuen wirtschaftlich und ökologisch besonders günstigen Verfahren können Farbstoffe der Formel

D-N=N-Z (I)

worin
D für den Rest einer aromatischen oder heterocyclischen Diazokomponente und
Z für den Rest einer Arylaminogruppe oder den Rest einer Heterocyclyliminogruppe der Formel (II) steht,

-N=Het (II)

worin
Het für ein heteroyclisches Diradikal steht,
hergestellt werden, indem man eine aromatische oder heterocycliche Diazokomponente der Formel

$D\text{-}NH_2$ (III)

und eine Verbindung der Formel

$H\text{-}Z^1$ (IV)

worin
$Z^1$ für Arylamino oder Hetarylamino steht,
in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umsetzt.

EP 0 567 849 A1

Die vorliegende Erfindung betrifft ein Verfahren zur Herstellung von Farbstoffen der allgemeinen Formel (I)

D-N=N-Z (I)

worin
D für den Rest einer aromatischen oder heterocyclischen Diazokomponente und
Z für den Rest einer Arylaminogruppe oder den Rest einer Heterocycyliminogruppe der Formel (II)

-N=Het (II)

steht, worin
Het für ein heterocyclisches Diradikal steht,
dadurch gekennzeichnet, daß eine aromatische oder heterocyclische Diazokomponente der Formel (III)

$D-NH_2$ (III)

und eine Verbindung der Formel (IV)

$H-Z^1$ (IV)

worin
$Z^1$ für Arylamino oder Hetarylamino steht,
in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

Vorzugsweise wird bei der Durchführung des erfindungsgemäßen Verfahrens auf den Zusatz weiterer Hilfsstoffe wie Emulgatoren, Säuren wie z.B. Salz-, Schwefel-, Salpeter-, Phosphor-, Ameisen-, Essig- oder Propionsäure oder Basen wie z.B. Alkalihydroxide oder -alkoholate verzichtet.

Als Reaktionsmedium eignet sich Wasser gegebenenfalls in Mischung mit organischen Lösungsmitteln, die ganz oder teilweise mit Wasser mischbar sind. Als geeignete Lösungsmittel kommen Methanol, Ethanol, Propanol, Isopropanol, Isoamylalkohol, Ethylenglykol, Methylglykol, Ethylglykol, Butylglykol, Ethylenglykoldimethylether, Ethylenglykoldiethylether, Diethylenglykoldimethylether, Glykoldiacetat, Methylglykolacetat, Ethylglykolacetat, Butylglykolacetat, Propylenglykol, Propylenglykolmonomethylether, Propylenglykolmonoethylether, Propylenglykoldimethylether, Propylenglykoldiethylether, Propylenglykolacetat, Triacetin, Acetonitril, Tetrahydrofuran, Dioxan, Dimethylformamid und N-Methylpyrrolidon in Frage. Vorzugsweise wird Wasser oder ein Gemisch aus Wasser und Methanol eingesetzt, das ein Verhältnis von Wasser zu Methanol von vorzugsweise 1:0,2 bis 1:10, besonders bevorzugt von 1:1 bis 1:5 und insbesondere von 1:2 aufweist.

Die Reaktion wird im allgemeinen bei 0 bis 125 °C durchgeführt. Vorzugsweise wird die Reaktion bei einer Temperatur von 0 bis 100 °C, insbesondere bei 0 bis 70 °C und ganz besonders bevorzugt bei 30 bis 40 °C durchgeführt.

Bei Normaldruck findet eine Reaktion mit $CO_2$ nicht statt.

Überraschenderweise wurde gefunden, daß bei Erhöhung des $CO_2$-Drucks auf über 5 bar die Diazotierungs- und Simultankupplungsreaktion zu Triazen- und Triazatrimethinfarbstoffen stattfindet. Der bevorzugte Druckbereich liegt bei 25 bis 65 bar.

Als salpetrige Säure abgebende Substanzen sind insbesondere anorganische und organische Nitrite geeignet.
Von den anorganischen Nitriten kommen vorzugsweise die Salpetrigsäuresalze der Elemente der ersten, zweiten und dritten Hauptgruppe des Periodensystems sowie der Übergangsmetalle in Frage, wie z.B. Lithiumnitrit, Natriumnitrit, Kaliumnitrit, Magnesiumnitrit, Calciumnitrit, Aluminiumnitrit, Eisennitrit, oder Kupfernitrit.

Von den organischen Nitriten sind die Salpetrigsäureester der Formel

E - O - N = O,

worin
E für einen gegebenfalls durch OH, -OAlkyl, -OAcetyl oder -ONO substituierten $C_1$-$C_{10}$-Alkylrest steht,
bevorzugt. Beispielhaft seien die folgenden organischen Nitrite genannt:

Methylnitrit, Ethylnitrit, n-Propylnitrit, i-Propylnitrit, n-Butylnitrit, i-Butylnitrit, s-Butylnitrit, n-Pentylnitrit, i-Pentylnitrit, alle Isomeren der Hexyl-, Heptyl- und Octylnitrit; 2-Methoxy-ethythylnitrit, 2-Ethoxyethylnitrit, 2-Propoxyethylnitrit, 2-Butoxyethylnitrit, 1-Methoxyprop-2-ylnitrit; weiterhin Salpetrigsäure Mono- und Diester von 1,2-Ethandiol, 1,2-Propandiol, 1,3-Propandiol, 1,4-Butandiol, 1,5-Pentandiol, 1,6-Hexandiol, 2-Methyl-1,3-propandiol, 2,2-Dimethyl-1,3-propandiol, 2,2-Diethyl-1,3-propandiol, 1-Propyl-2-ethyl-1,3-propandiol, 1-Propyl-2,2-dimethyl-1,3-propandiol, 2-Ethyl-2-butyl-1,3-propandiol; Diethylenglykol, Di-1,2-propylenglykol und Triethylenglykol sowie der Methyl-, Ethyl-, Propyl-und Butylhalbether von Diethylenglykol, Di-1,2-Propylenglykol und Triethylenglykol, sowie die Salpetrigsäureester des Pentaerythrits und der daraus abgeleiteten Alkylether.

Vorzugsweise wird die Reaktion während einer Zeit von 10 Minuten bis 24 Stunden durchgeführt.

Die Herstellung von Triazen- und Triazatrimethinfarbstoffen wurde bisher durch Diazotieren der aromatischen oder heterocyclischen Amine in mineralsaurer Lösung, Zugabe der Diazoniumsalzlösung zur Lösung der Kupplungskomponente und Kuppeln durch Zugabe von säurebindenden Mitteln durchgeführt (vgl. DE-A 30 45 912 und DE-A 10 69 563). In jedem Fall wurde bisher eine anorganische oder organische Säure zur Diazotierung eingesetzt, die bei der anschließenden Kupplung neutralisiert werden mußte. Hierbei entstehen anorganische Salze. Diese anorganischen Salze gelangen bei der Isolierung des Farbstoffes in das Abwasser und stellen eine Belastung dar. In Mischung mit dem hergestellten Farbstoff beeinflußen anorganische Salze häufig die Löslichkeit in nachteiliger Weise. Zur Herstellung stabiler Farbstofflösungen müssen diese Salze durch aufwendige Verfahren, beispielsweise Druckpermeation oder Reversosmose entfernt werden. Alle diese Nachteile können durch das neue erfindungsgemäße Herstellungsverfahren vermieden werden,

Das erfindungsgemäße Verfahren dient vorzugsweise zur Herstellung von Farbstoffen der Formel (I)

D-N = N-Z (I)

worin

D für den Rest einer substituierten oder unsubstituierten aromatischen Diazokomponente mit 6 bis 10 Kohlenstoffatomen oder für den Rest einer substituierten oder unsubstituierten 5- oder 6-gliedrigen heterocyclischen Diazokomponente, die 1 bis 3 Heteroatome aus der Reihe Sauerstoff, Schwefel, Stickstoff enthält und gegebenenfalls einen oder zwei anellierte 5- und/oder 6-gliedrige substituierte oder unsubstituierte carbocyclische Ringe enthält, steht,

Z für den Rest einer substituierten oder unsubstituierten $C_6$-$C_{10}$-Arylaminogruppe oder für den Rest einer substituierten oder unsubstituierten Heterocyclyliminogruppe der Formel (II)

-N = Het (II)

steht, worin

Het für das Diradikal eines substituierten oder unsubstituierten gesättigten oder partiell ungesättigten 5- oder 6-gliedrigen heterocyclischen Rings, der 1 bis 3 Heteroatome aus der Reihe Sauerstoff, Schwefel, Stickstoff enthält und gegebenenfalls durch ein oder zwei 5- und/oder 6-gliedrige substituierte oder unsubstituierte carbocyclische Ringe anelliert ist, steht,

indem eine aromatische oder heterocyclische Diazokomponente der Formel (III), worin

D die oben angegebene Bedeutung hat,

und eine Verbindung der Formel (IV), worin

$Z^1$ für den Rest einer substituierten oder unsubstituierten $C_6$-$C_{10}$-Arylaminogruppe oder für den Rest einer substituierten oder unsubstituierten 5- oder 6-gliedrigen Hetarylaminogruppe die 1 bis 3 Heteroatome aus der Reihe Sauerstoff, Schwefel, Stickstoff im Hetarylteil enthält und gegebenenfalls durch einen oder zwei 5- und/oder 6-gliedrige substituierte oder unsubstituierte carbocyclische Ringe anelliert ist, steht

und wobei als Substituenten für die substituierten aromatischen Diazokomponenten, die substituierten 5- oder 6-gliedrigen heterocyclischen Diazokomponenten, den Arylteil der substituierten $C_6$-$C_{10}$-Arylaminogruppen in der Definition von Z und $Z^1$, den Heterocyclylteil der substituierten Heterocyclyliminogruppen der Formel (II), den Hetarylteil der substituierten Hetarylaminogruppen und die substituierten carbocyclischen Ringein der Definition von D, Het und $Z^1$ in Frage kommen: $C_1$-$C_8$-Alkyl, $C_6$-$C_{10}$-Aryl, $C_6$-$C_{10}$-Aryloxy, $C_6$-$C_{10}$-Aryl-$C_1$-$C_4$-alkyl, $C_6$-$C_{10}$-Aryl-$C_1$-$C_4$-alkyloxy, $C_2$-$C_4$-Alkenyl, $C_1$-$C_4$-Alkoxy, $C_1$-$C_4$-Alkoxycarbonyl, $C_1$-$C_4$-Alkylcarbonyloxy, Cyano, $C_1$-$C_4$-(Di)alkylamino, $C_1$-$C_4$-(Di)alkylaminocarbonyl, $C_1$-$C_4$-Alkylcarbonylamino, Hydroxy, Halogen, $C_1$-$C_4$-Alkylcarbonyl, $C_1$-$C_4$-Alkylsulfonyl, $C_1$-$C_4$-(Di)-alkylaminosulfonyl, $C_1$-$C_4$-Alkylsulfonylamino, $C_1$-$C_4$-Alkoxysulfonyl, $C_1$-$C_4$-Alkylsulfonyloxy, Nitro, $C_6$-$C_{10}$-(Di)arylamino, $C_1$-$C_4$-

Alkyl-$C_6$-$C_{10}$-arylamino, Phenylsulfonyl, Phenylazo, Benzothiazolyl, 1,2,4-Oxadiazolyl, Morpholino, Piperidino, Piperazino, Pyrrolidino, $C_1$-$C_4$-Alkylmercapto und $C_6$-$C_{10}$-Arylmercapto, und wobei für den Aminoteil der substituierten $C_6$-$C_{10}$- Arylaminogruppen in der Definition von Z und $Z^1$ als Substituenten gegebenenfalls substituiertes $C_1$-$C_8$-Alkyl, $C_2$-$C_4$-Alkenyl, substituiertes oder unsubstituiertes $C_1$-$C_4$-Alkyl-$C_6$-$C_{10}$-aryl in Frage kommen,
in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

Besonders bevorzugterweise dient das erfindungsgemäße Verfahren zur Herstellung von Farbstoffen der Formel (I),
worin

D für den Rest einer substituierten oder unsubstituierten aromatischen 6 bis 10 Kohlenstoffatome enthaltenden Diazokomponente oder für den Rest einer heterocyclischen Diazokomponente aus der Reihe der jeweils gegebenenfalls substituierten und jeweils gegebenenfalls benzanellierten Thiazole, Isothiazole, Thiadiazole, Oxazole, Imidazole und Triazole steht, und

Z für einen Rest der Formel

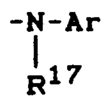

(V)

oder -N = Het (II)

steht, worin

Ar für jeweils gegebenenfalls substituiertes Phenyl oder Naphthyl steht,

$R^{17}$ für Wasserstoff, gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiertes $C_1$-$C_8$-Alkyl steht oder für $C_2$-$C_4$-Alkylen, für jeweils gegebenenfalls durch Halogen, Cyano, $C_1$-$C_4$-Alkyl oder $C_1$-$C_4$-Alkoxy substituiertes Phenylmethyl oder Phenylethyl steht,

Het für das Diradikal eines partiell ungesättigten, 5-gliedrigen, 1 bis 3 Heteroatome aus der Reihe Sauerstoff, Schwefel, Stickstoff enthaltenden heterocyclischen Rings, der gegebenenfalls durch einen substituierten oder unsubstituierten Benzolring anelliert ist steht, wobei der heterocyclische Ring gegebenenfalls 1- bis 3-fach gleich oder verschieden substituiert ist durch $C_1$-$C_8$-Alkyl, das gegebenenfalls durch Hydroxy, Halogen, Cyano, $C_1$-$C_4$-Alkoxy, Carboxyl, $C_1$-$C_4$-(Di)-alkylaminocarbonyl, $C_1$-$C_4$-Alkoxycarbonyl oder $C_1$-$C_4$-Alkylsulfonyl substituiert ist, weiterhin durch $C_2$-$C_4$-Alkenyl, oder durch Phenylmethyl oder Phenylethyl, welches jeweils gegebenenfalls durch Halogen, Cyano, $C_1$-$C_4$-Alkyl, $C_1$-$C_4$-Alkoxy, $C_1$-$C_4$-(Di)alkylamino, $C_6$-$C_{10}$-(Di)arylamino, $C_1$-$C_4$-Alkyl-$C_6$-$C_{10}$-arylamino, Morpholino, Piperidino, Piperazino, Pyrrolidino, $C_1$-$C_4$-Alkylmercapto oder $C_6$-$C_{10}$-Aryl-mercapto substituiert ist,

indem eine aromatische oder heterocyclische Diazokomponente der Formel (III), worin

D die oben angegebene Bedeutung hat,

und eine Verbindung der Formel (IV), worin

$Z^1$ für einen Rest der Formel

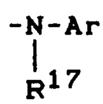

(V)

oder

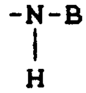

(VI)

steht, worin

Ar und $R^{17}$ die oben angegebene Bedeutung haben, und

B für den Rest eines 5-gliedrigen ungesättigten heterocyclischen Rings steht, der 1 bis 3 Heteroatome aus der Reihe Sauerstoff, Schwefel, Stickstoff enthält und der gegebenenfalls durch einen substituierten oder unsubstituierten Benzolring anelliert ist, wobei der heterocyclische Ring gegebenenfalls 1- bis 3-fach gleich oder verschieden substituiert ist durch $C_1$-$C_8$- Alkyl, welches gegebenenfalls durch Hydroxy, Halogen, Cyano, $C_1$-$C_4$-Alkoxy, Carboxyl, $C_1$-$C_4$-(Di)-alkylaminocarbonyl, $C_1$-$C_4$-Alkoxycarbonyl oder $C_1$-$C_4$-Alkylsulfonyl substituiert ist, durch $C_2$-$C_4$-Alkenyl oder durch Phenylmethyl oder Phenylethyl, welches jeweils gegebenenfalls durch Halogen, Cyano, $C_1$-$C_4$-Alkyl oder $C_1$-$C_4$-Alkoxy, $C_1$-$C_4$-(Di)alkylamino, $C_6$-$C_{10}$-(Di)arylamino, $C_1$-$C_4$-Alkyl-$C_6$-$C_{10}$-arylamino, Morpholino, Piperidino, Piperazino, Pyrrolidino, $C_1$-$C_4$-Alkylmercapto oder $C_6$-$C_{10}$-Arylmercapto substituiert ist,

und wobei als Substituenten für die substituierten aromatischen Diazokomponenten und die substituierten Phenyl- und Naphthyl-Reste in der Definition von Z und $Z^1$ und die anellierten Benzolringe in der Definition von Het und B in Frage kommen:

$C_1$-$C_8$-Alkyl, $C_6$-$C_{10}$-Aryl, $C_6$-$C_{10}$-Aryloxy, $C_6$-$C_{10}$-Aryl-$C_1$-$C_4$-alkyl-, $C_6$-$C_{10}$-Aryl-$C_1$-$C_4$-alkyloxy, $C_2$-$C_4$-Alkenyl, $C_1$-$C_4$-Alkoxy, $C_1$-$C_4$-Alkoxycarbonyl, $C_1$-$C_4$-Alkylcarbonyloxy, Cyano, $C_1$-$C_4$-(Di)alkylamino, $C_1$-$C_4$-(Di)alkylaminocarbonyl, $C_1$-$C_4$-Alkylcarbonylamino, Hydroxy, Halogen, $C_1$-$C_4$-Alkylcarbonyl, $C_1$-$C_4$-Alkylsulfonyl, $C_1$-$C_4$-(Di)alkylaminosulfonyl, $C_1$-$C_4$-Alkylsulfonylamino, $C_1$-$C_4$-Alkoxysulfonyl, $C_1$-$C_4$-Alkylsulfonyloxy, Nitro, $C_6$-$C_{10}$-(Di)arylamino, $C_1$-$C_4$-Alkyl-$C_6$-$C_{10}$-arylamino, Phenylsulfonyl, Phenylazo, Benzothiazolyl, 1,2,4-Oxadiazolyl, Morpholino, Piperidino, Piperazino, Pyrrolidino, $C_1$-$C_4$-Alkylmercapto und $C_6$-$C_{10}$-Arylmercapto, und wobei für den Aminoteil der substituierten $C_6$-$C_{10}$- Arylaminogruppen in der Definition von Z und $Z^1$ als Substituenten gegebenenfalls substituiertes $C_1$-$C_8$-Alkyl, $C_2$-$C_4$-Alkenyl, substituiertes oder unsubstituiertes $C_1$-$C_4$-Alkyl-$C_6$-$C_{10}$-aryl in Frage kommen,

in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

Insbesondere dient das erfindungsgemäße Verfahren zur Herstellung von Farbstoffen der Formel (I), worin

D für einen Rest der Formel

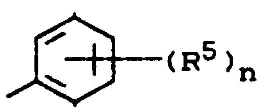 ,

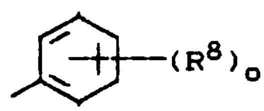 ,

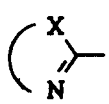

oder

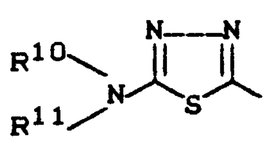

steht, worin

$R^5$ für Wasserstoff, Halogen $C_1$-$C_4$-Alkyl, $C_1$-$C_4$-Alkoxy, Benzyl, Benzyloxy, für jeweils gegebenenfalls durch Halogen, $C_1$-$C_4$-Alkyl, $C_1$-$C_4$-Alkoxy, Acetyl, Benzoyl oder $C_1$-$C_4$-Alkoxycarbonyl substituiertes Phenyl oder Phenyloxy oder für Phenylazo steht, oder

$R^5$ zusammen mit dem Benzolring an den es gebunden ist für ein Tetralin-, Naphthalin- oder Benzodioxan-System steht,

n für 1 oder 2 steht,

$R^8$ für Wasserstoff, Halogen, $C_1$-$C_4$-Alkyl, $C_1$-$C_4$-Alkoxy, $C_1$-$C_4$-(Di)alkylamino, $C_1$-$C_4$-(Tri)-alkyl-ammonium, Benzyl, Benzyloxy, Phenyloxy, Acetyl, Benzoyl, $C_1$-$C_4$-Alkoxycarbonyl,

Carboxamido, Carboxyl, Nitro, Sulfonamido oder Sulfo steht,

o für 1 oder 2 steht,

X für die restlichen Glieder eines Thiazol-, Isothiazol-, Benzthiazol-, 1,2,4-Triazol- oder 1,3,4-Thiadiazolrings steht,

$R^{10}$ und $R^{11}$ unabhängig voneinander für Wasserstoff oder für gegebenenfalls durch Halogen, OH, Cyano, $C_1$-$C_4$-Alkoxy, $C_1$-$C_4$-Carbonyloxy, Phenoxy oder Phenyl substituiertes $C_1$-$C_4$-Alkyl stehen,

oder

$R^{10}$ und $R^{11}$ zusammen mit dem Stickstoffatom an das sie gebunden sind für die restlichen Glieder eines 5-oder 6-gliedrigen heterocyclischen Rings, der ein weiteres Heteroatom aus der Reihe Sauerstoff, Schwefel, Stickstoff enthalten kann, stehen,

$R^{10}$ weiterhin gegebenenfalls durch Halogen, $C_1$-$C_4$-Alkyl oder $C_1$-$C_4$-Alkoxy substituiertes Phenyl steht,

und

$R^{11}$ weiterhin für einen Alkylenrest steht, der durch Ringschluß mit dem Thiadiazolring einen partiell ungesättigten 5- oder 6-gliedrigen heterocyclischen Ring bildet,

Z für den Rest der Formel

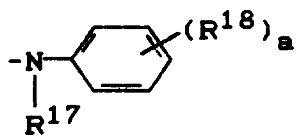

,

(VII)

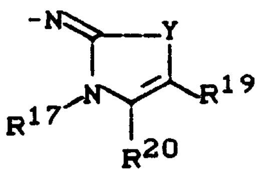

,

(VIII)

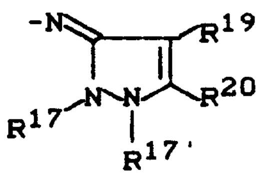

,

(IX)

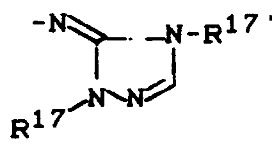

,

(X)

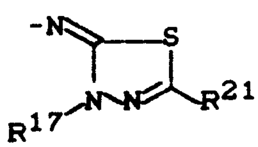

(XI)

oder

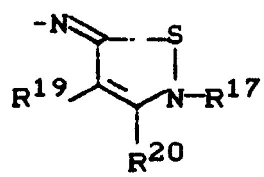

(XII)

steht, worin

Y für -S-, -O- oder

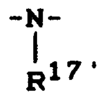

steht,
worin

$R^{17}$ und $R^{17'}$ unabhängig voneinander für Wasserstoff, gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiertes $C_1$-$C_8$-Alkyl steht, oder für $C_2$-$C_4$-Alkenyl, oder für jeweils gegebenenfalls durch Halogen, Cyano, $C_1$-$C_4$-Alkyl oder $C_1$-$C_4$-Alkoxy substituiertes Phenylmethyl oder Phenylethyl stehen,

$R^{18}$ für $C_1$-$C_4$-Alkyl, $C_1$-$C_4$-Alkoxy, Halogen, Nitro, Cyano, $C_1$-$C_4$-Alkylsulfonyl, Phenylsulfonyl, $C_1$-$C_4$-Alkylcarbonylamino, Phenylazo, Benzothiazolyl oder 1,2,4-Oxadiazolyl steht,

a für 0, 1, 2 oder 3 steht,

$R^{19}$ und $R^{20}$ entweder für Wasserstoff stehen oder gemeinsam für die restlichen Glieder eines anellierten Benzolrings stehen und

$R^{21}$ für $C_1$-$C_4$-(Di)alkylamino, $C_6$-$C_{10}$-(Di)arylamino, $C_1$-$C_4$-Alkyl-$C_6$-$C_{10}$-arylamino, Morpholino, Piperidino, Piperazino, Pyrrolidino, $C_1$-$C_4$-Alkylmercapto oder $C_6$-$C_{10}$-Arylmercapto steht,

indem eine aromatische oder heterocyclische Diazokomponente der Formel (III), worin

D die oben angegebene Bedeutung hat,

und eine Verbindung der Formel (IV), worin

$Z^1$ für einen Rest der Formel

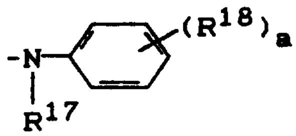

,

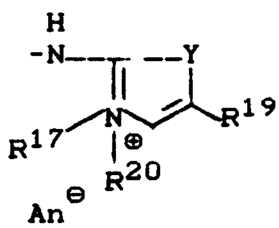

,

(XIII) (XIV)

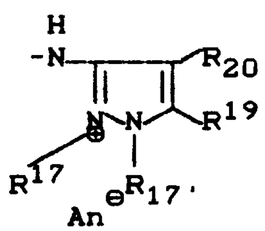

,

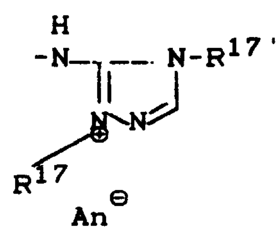

,

(XV) (XVI)

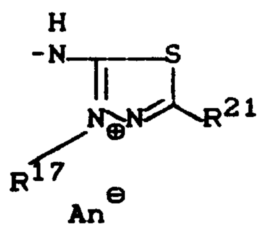

(XVII)

oder

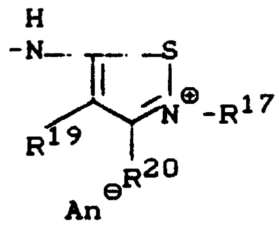

(XVIII)

steht, worin

$Y$, $R^{17}$, $R^{17'}$, $R^{18}$, $R^{19}$, $R^{20}$, $R^{21}$ und a die oben angegebene Bedeutung haben und

$An^{\ominus}$ für ein Anion aus der Reihe $Cl^{\ominus}$, $CH_3OSO_2O^{\ominus}$, $I^{\ominus}$, $Br^{\ominus}$, $CH_3COO^{\ominus}$ steht,

in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

Alle in den obigen Definitionen als Substituenten genannten Alkyl-, Alkenyl-, Alkoxy- und Arylreste, auch wenn sie in zusammengesetzten Begriffen wie z.B. Alkylcarbonyl oder Aryloxy auftreten, können generell durch übliche Substituenten wie z.B. Halogen, Hydroxy, Cyano, $C_1$-$C_4$-Alkoxy, Carboxyl, Aminocarbonyl, $C_1$-$C_4$-(Di)alkylamino, $C_1$-$C_4$-(Di)alkylaminocarbonyl, $C_1$-$C_4$-Alkoxycarbonyl, $C_1$-$C_4$-Alkylsulfonyl, Nitro weiter substituiert sein.

Als Substituenten für die oben genannten anellierten Benzolringe kommen generell infrage: $C_1$-$C_4$-Alkyl, $C_1$-$C_4$-Alkoxy, Nitro, Chlor, Brom, Cyano, $C_1$-$C_4$-Alkoxycarbonyl und $C_1$-$C_4$-Alkoxysulfonyl.

Gemäß dem erfindungsgemäßen Verfahren können insbesondere Farbstoffe der Formel

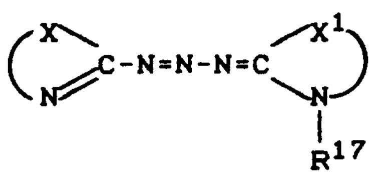

(XIX)

worin

- X für die restlichen Glieder eines Thiazol-, Isothiazol-, Benzthiazol-, 1,2,4-Triazol- oder 1,3,4-Thiadiazolrings steht,
- $X^1$ für die restlichen Glieder eines Thiazolin-, Isothiazolin-, Benzthiazolin-, 1,2,4-Triazolin- oder 1,3,4-Thiadiazolinrings steht,
- $R^{17}$ für gegebenenfalls durch Cyano, $C_1$-$C_4$-(Di)alkylaminocarbonyl, Hydroxyl, $C_1$-$C_4$-Alkoxy, Carboxyl, $C_1$-$C_4$-Alkoxycarbonyl oder $C_1$-$C_4$-Alkylsulfonyl substituiertes $C_1$-$C_4$-Alkyl steht,

hergestellt werden, indem ein heterocyclisches Amin der Formel

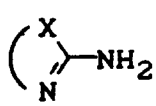

(XX)

worin X die oben angegebene Bedeutung hat,
und eine heterocyclische Ammoniumverbindung der Formel

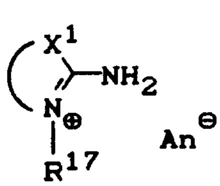

(XXI)

worin

$X^1$ und $R^{17}$ die oben angegebene Bedeutung haben und

An$^{\ominus}$ für ein Anion aus der Reihe $Cl^{\ominus}$, $CH_3OSO_2^{\ominus}$, $I^{\ominus}$, $Br^{\ominus}$,$CH_3COO^{\ominus}$ steht,

in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

Beispiele für Verbindungen der Formel (IV), worin $Z_1$ für einen Rest der Formel (V) steht sind: Anilin, p-Toluidin, p-Anisidin, N-Methylanilin, N-Ethylanilin, N-Propylanilin, N-Butylanilin, N-Cyanethylanilin, N-Hydroxyethylanilin, N-Hydroxypropylanilin, N-Amidoethylanilin, N-Methyl-2-toluidin, N-Methyl-3-toluidin, N-Methyl-4-toluidin, N-Cyanethyl-4-toluidin, N-Methyl-2-anisidin, N-Methyl-3-anisidin, N-Methyl-2-anisidin, N-Allylanilin, N-Benzylanilin, N-Phenethyl-p-toluidin.

Beispiele für Verbindungen der Formel (XX) sind:
2-Amino-3-methylbenzthiazol, 2-Amino-3-ethylbenzthiazol, 3-Amino-3-methyl-6-methylbenzthiazol, 2-Amino-3-methyl-6-methoxybenzthiazol, 2-Amino-3-methyl-6-nitrobenzthiazol, 2-Amino-3-methyl-6-ethoxybenzthiazol, 2-Amino-3--methyl-1,3-thiazol, 2-Amino-3-methyl-5-methyl-1,3-thiazol, 2-Amino-3-methyl-5-ethyl-1,3-thiazol, 2-Amino-3-methyl-5-t-butyl-1,3-thiazol, 2-Amino-3-methyl-5-phenyl-1,3-thiazol, 2-Amino-3-methyl-4,5-dimethyl-1,3-thiazol, 2-Amino-3-methyl-4,5-diphenyl-1,3-thiazol, 3-Aminoisothiazol, 3-Amino-1,4-dimethyl-1,2,4-triazol, 3-Amino-1,4-diethyl-1,2,4-triazol, 3-Amino-1,4-dimethyl-5-ethyl-1,2,4-triazol, 3-Amino-1,4-dimethyl-5-cyanethyl-1,2,4-triazol, 3-Amino-1,4-dimethyl-5-butyl-1,2,4-triazol, 3-Amino-1,4-dimethyl-5-phenyl-1,2,4-triazol, 3-Amino-1,4-dimethyl-5-p-tolyl-1,2,4-triazol, 3-Amino-1,2,4-triazol-5-carbonsäure, 2-Amino-1,3,4-thiadiazol, 2-Amino-5-methyl-1,3,4-thiadiazol, 2-Amino-5-n-propyl-1,3,4-thiadiazol, 2-Amino-5-i-propyl-1,3,4-thiadiazol, 2-Amino-5-(di)methylamino-1,3,4-thiadiazol, 2-Amino-5-(di)ethylamino-1,3,4-thiadiazol, 2-Amino-5-(di)-n-propylamino-1,3,4-thiadiazol, 2-Amino-5-(di)-i-propylamino-1,3,4-thiadiazol, 2-Amino-5-(di)-n-butylamino-1,3,4-thiadiazol, 2-Amino-5-(di)-phenylamino-1,3,4-thiadiazol, 2-Amino-5-phenylmethylamino-1,3,4-thiadiazol, 2-Amino-5-benzylmethylamino-1,3,4-thiadiazol, 2-Amino-5-phenethylmethylamino-1,3,4-thiadiazol, 2-Amino-5-morpholino-1,3,4-thiadiazol, 2-Amino-5-piperidino-1,3,4-thiadiazol, 2-Amino-5-pyrrolidino-1,3,4-thiadiazol, 2-Amino-5-(di)cyclohexylamino-1,3,4-thiadiazol, 2-Amino-5-cyclohexylmethylamino-1,3,4-thiadiazol, 2-Amino-5-(di)hydroxyethylmethylamino-1,3,4-thiadiazol, 2-Amino-5-cyanethylmethylamino-1,3,4-thiadiazol.

Beispiele für Verbindungen der Formel (XXI) sind:
2-Amino-3-methylbenzthiazolium-, 2-Amino-3-ethylbenzthiazolium-, 2-Amino-3-methyl-6-methylbenzthiazolium-, 2-Amino-3-methyl-6-methoxybenzthiazolium-, 2-Amino-3-methyl-6-nitrobenzthiazolium -, 2-Amino-3-methyl-6-ethoxybenzthiazolium-, 2-Amino-3-methyl-1,3-thiazolium-, 2-Amino-3-methyl-5-methyl-1,3-thiazolium-, 2-Amino-3-methyl-5-ethyl-1,3-thiazolium-, 2-Amino-3-methyl-5-t-butyl-1,3-thiazolium-, 2-Amino-3-methyl-5-phenyl-1,3-thiazolium-, 2-Amino-3-methyl-4,5-dimethyl-1,3-thiazolium-, 2-Amino-3-methyl-4,5-diphenyl-1,3-thiazolium-, 3-Amino-1,4-dimethyl-1,2,4-triazolium-, 3-Amino-1,4-diethyl-1,2,4-triazolium-, 3-Amino-1,4-dimethyl-5-ethyl-1,2,4-triszolium-, 3-Amino-1,4-dimethyl-5-cyanethyl-1,2,4-triazolium-, 3-Amino-1,4-dimethyl-5-butyl-1,2,4-triazolium-, 3-Amino-1,4-dimethyl-5-phenyl-1,2,4-triazolium-, 3-Amino-1,4-dimethyl-5-p-tolyl-1,2,4-triazolium-, 2-Amino-1,3,4-thiadiazolium-, 2-Amino-5-methyl-1,3,4-thiadiazolium-,2-Amino-5-(di)methylamino-1,3,4-thiadiazolium-, 2-Amino-5-(di)ethylamino-1,3,4-thiadiazolium-, 2-Amino-5-(di)-n-propylamino-1,3,4-thiadiazolium-, 2-Amino-5-(di)-i-propylamino-1,3,4-thiadiazolium-, 2-Amino-5-(di)-n-butylamino-1,3,4-thiadiazolium-, 2-Amino-5-(di)-phenylamino-1,3,4-thiadiazolium-, 2-Amino-5-phenylmethylamino-1,3,4-thiadiazolium-, 2-Amino-5-benzylmethylamino-1,3,4-thiadiazolium-, 2-Amino-5-phenethylmethylamino-1,3,4-thiadiazolium-, 2-Amino-5-morpholino-1,3,4-thiadiazolium-, 2-Amino-5-piperidino-1,3,4-thiadiazolium-, 2-Amino-5-pyrrolidino-1,3,4-thiadiazolium-, 2-Amino-5-(di)cyclohexylamino-1,3,4-thiadiazolium-, 2-Amino-5-cyclohexylmethylamino-1,3,4-thiadiazolium-, 2-Amino-5-(di)-hydroxyethylmethylamino-1,3,4-thiadiazolium-, 2-Amino-5-cyanethylmethylamino-1,3,4-thiadiazoliumsalze.

Weitere Beispiele für Verbindungen der Formel (XXI) können durch Quaternierung der oben beispielhaft aufgeführten Verbindungen der Formel (XX) mit den im folgenden aufgeführten Alkylierungsmitteln erhalten werden:
Methyliodid, Ethyliodid, Propyliodid, Butyliodid, Dimethylsulfat, Ethylenoxid, Propylenoxid, Acrylnitril, Acrylamid, Acrylsäure und ihre Ester, $\alpha$-Halogencarbonsäuren und ihre Ester, Epichlorhydrin.

Beispiel 1

In einem Autoklaven werden 20 g 2-Amino-5-diisopropylamino-1,3,4-thiadiazol, 27,6 g 2-Amino-3-methyl-1,3-benzthiazoliummethosulfat, 12 g Isoamylnitrit, 40 ml Wasser und 80 ml Methanol vereinigt, unter einen $CO_2$-Druck von 50 bar gesetzt und auf 40 °C erwärmt. Bei dieser Temperatur wird unter konstantem Druck 3 Stunden gerührt. Dann wird der Autoklav entspannt, das Methanol abdestilliert und die Farbbase

der Formel

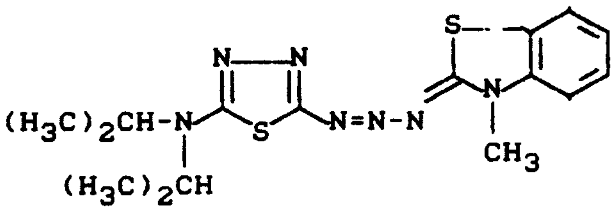

auf Wasser ausgetragen und abgesaugt. Nach dem Trocknen beträgt die Ausbeute 28,5 g, Fp: 195-198 ° C.

Durch Methylierung mit Dimethylsulfat erhält man einen Kationischen Farbstoff, der Polyacrylnitril in lichtechtem rotem Ton anfärbt und der Formel

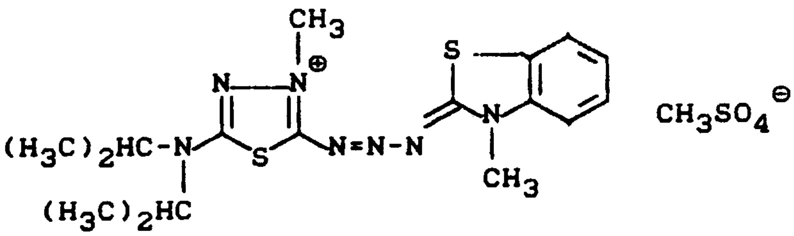

entspricht.

Die Reaktion läuft vermutlich nach folgendem Schema ab:

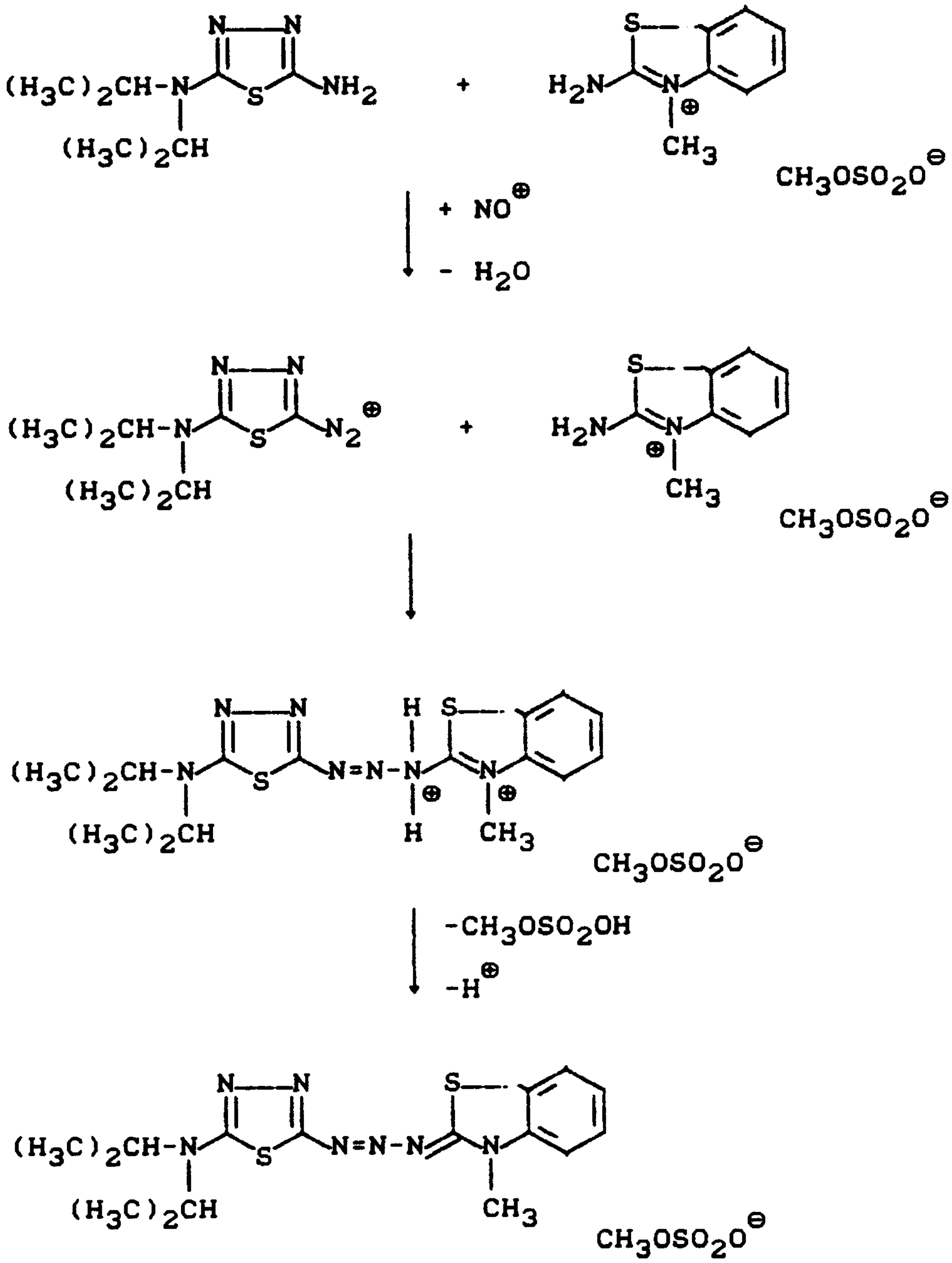


Ähnlich wertvolle Farbstoffe erhält man, wenn anstelle von 2-Amino-3-methyl-1,3-benzthiazoliummethosulfat die methylierten Quartärsalze von 3-Aminotriazol, von 2-Aminothiazol oder von 2-Amino-diisopropylamino-1,3,4-thiadiazol für die Synthese der Farbbase einsetzt.

Ersetzt man 2-Amino-5-diisopropylamino-1,3,4-thiadiazol durch 3-Aminotriazol, 2-Aminothiazol oder 2-Amino-1,3-benzthiazol, so erhält man ebenfalls Produkte, die nach Quaternierung günstige färberische Eigenschaften aufweisen.

Analog Beispiel 1 lassen sich die in Tabelle 1 aufgeführten Farbstoffe der Formel D-N = N-Z herstellen, die nach dem Quaternieren mit Q die angegebene Absorptionswellenlänge aufweisen:

**Tabelle 1**

| Bsp. Nr. | D-N=N-Z | Q | $\lambda_{max}$ (nm) |
|---|---|---|---|
| 2 | 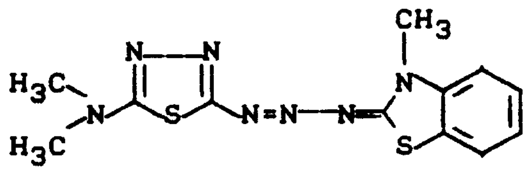 | Dimethyl-sulfat | 490 |
| 3 | 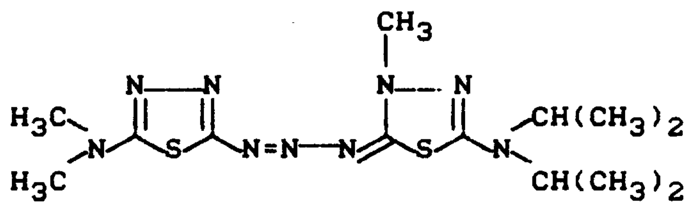 | Dimethyl-sulfat | 492 |
| 4 | 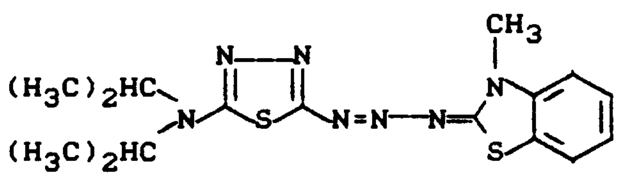 | Dimethyl-sulfat | 492 |
| 5 | 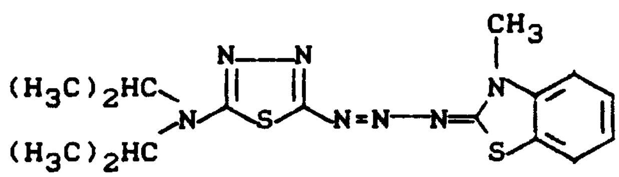 | Acryl-nitril | 465 |
| 6 | 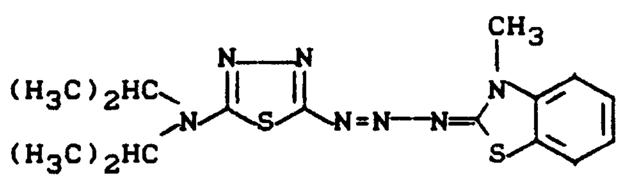 | Benzyl-chlorid | 498 |
| 7 | 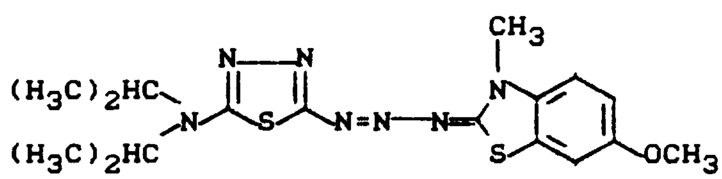 | Dimethyl-sulfat | 505 |

<u>Tabelle 1</u> - Fortsetzung

| Bsp. Nr. | D-N=N-Z | Q | $\lambda_{max}$ (nm) |
|---|---|---|---|
| 8 | 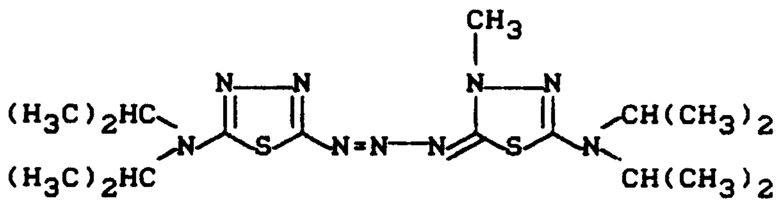 | Dimethylsulfat | 492 |
| 9 | 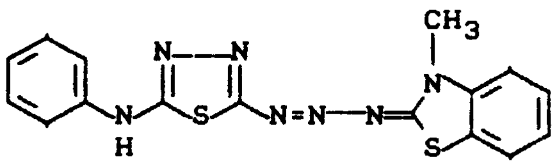 | Dimethylsulfat | 498 |
| 10 | 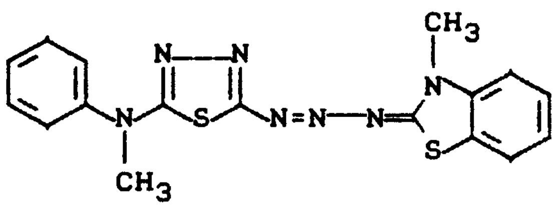 | Dimethylsulfat | 500 |
| 11 | 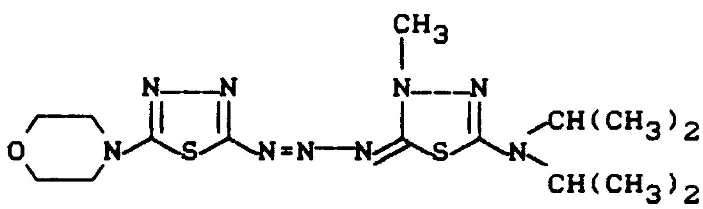 | Dimethylsulfat | 496 |
| 12 | 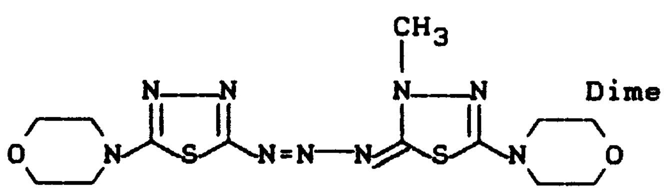 | Dimethylsulfat | 494 |

Tabelle 1 - Fortsetzung

| Bsp. Nr. | D-N=N-Z | Q | $\lambda_{max}$ (nm) |
|---|---|---|---|
| 13 | 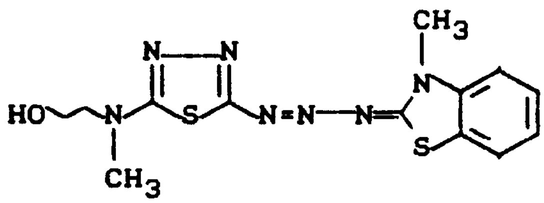 | Dimethylsulfat | 496 |
| 14 | 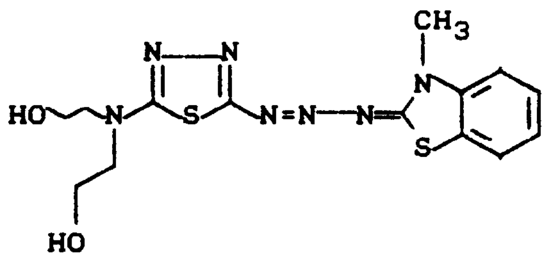 | Dimethylsulfat | 496 |
| 15 | 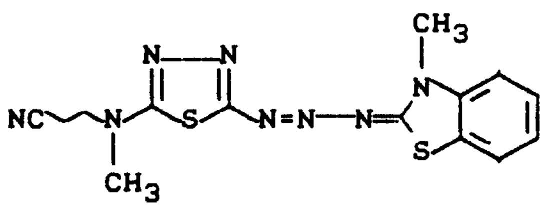 | Dimethylsulfat | 490 |
| 16 | 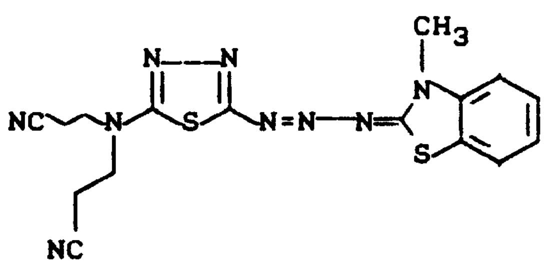 | Dimethylsulfat | 488 |
| 17 | 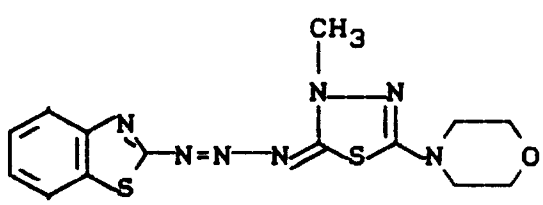 | Dimethylsulfat | 490 |

**Tabelle 1 - Fortsetzung**

| Bsp. Nr. | D-N=N-Z | Q | $\lambda_{max}$ (nm) |
|---|---|---|---|
| 18 | 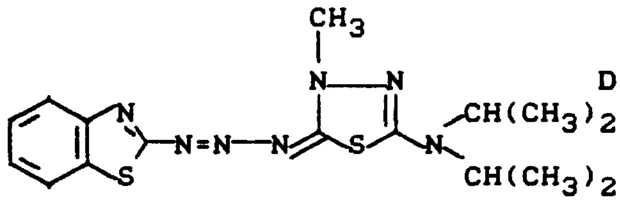 | Dimethylsulfat | 492 |
| 19 | 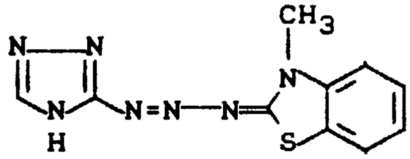 | Dimethylsulfat | 428 |
| 20 | 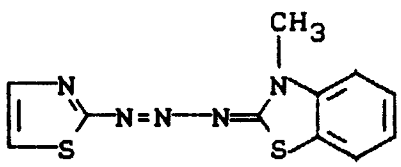 | Dimethylsulfat | 466 |
| 21 | 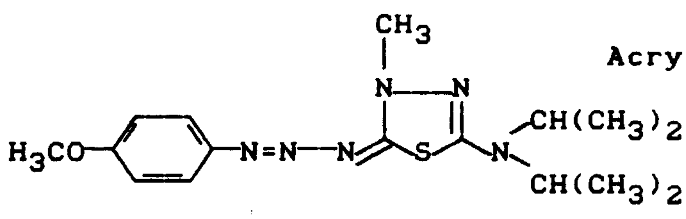 | Acrylnitril | 434 |

Beispiel 22

In einem Autoklaven werden 40 g 2-Amino-5-diisopropylamino-1,3,4-thiadiazol, 32 g N-Cyanethyl-4-toluidin, 14,6 g Natriumnitrit und 120 ml Wasser vereinigt und unter einen Kohlendioxiddruck von 50 bar gesetzt. Bei diesem Druck und 40 °C rührt man 3 Stunden. Der Autoklav wird entspannt und die Farbbase der Formel

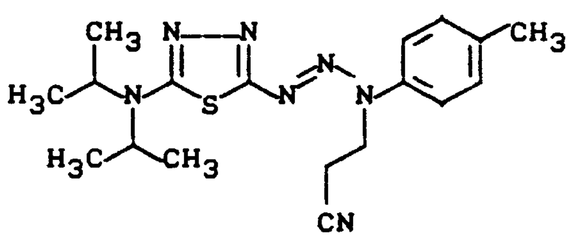

abfiltriert. Nach dem Trocknen beträgt die Ausbeute 69,8 g, entsprechend 94 % der Theorie, vom Fp.: 123 bis 127 °C.

Durch Quaternierung mit hierfür üblichen Reagentien erhält man kationische Farbstoffe, die Polyacrylnitril in lichtechten, gelben Tönen anfärben.

Analog zu Beispiel 21 wurden die folgenden Farbstoffe der Formel D-N = N-Z erhalten, die nach Quaternierung mit Q die in Tabelle 2 angegebenen $\lambda_{max}$-Werte aufweisen.

**Tabelle 2**

| Bsp. Nr. | D-N=N-Z | Q | $\lambda_{max}$ (nm) |
|---|---|---|---|
| 23 | 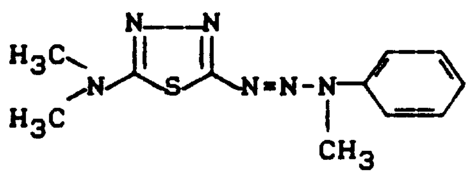 | Dimethylsulfat | 420 |
| 24 | 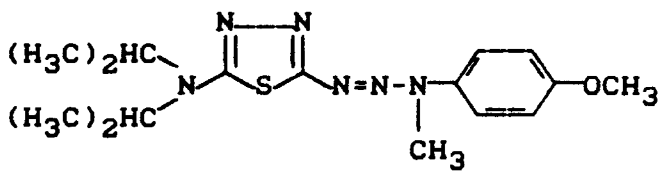 | Dimethylsulfat | 428 |
| 25 | 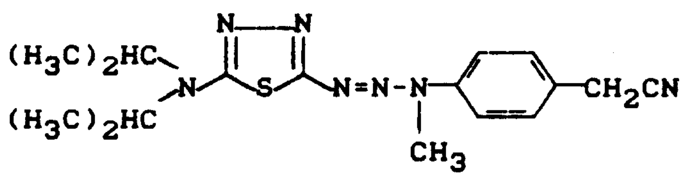 | Dimethylsulfat | 434 |
| 26 | 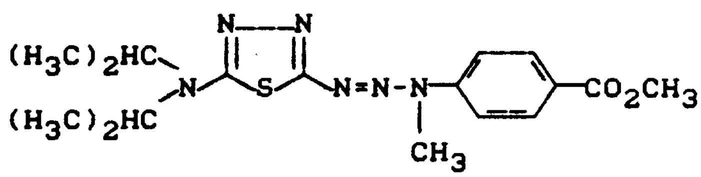 | Dimethylsulfat | 428 |
| 27 | 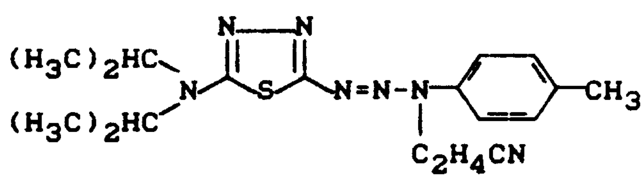 | Dimethylsulfat | 426 |
| 28 | 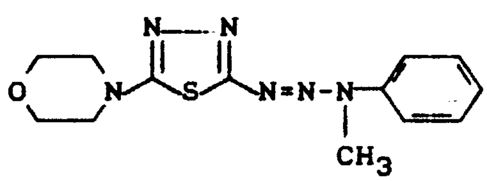 | Dimethylsulfat | 420 |

**Tabelle 2 - Fortsetzung**

| Bsp. Nr. | D-N=N-Z | Q | $\lambda_{max}$ (nm) |
|---|---|---|---|
| 29 | 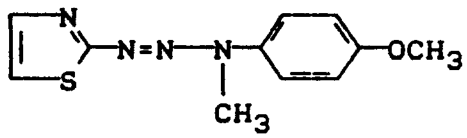 | Dimethylsulfat | 408 |
| 30 | 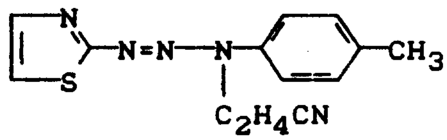 | Dimethylsulfat | 400 |
| 31 | 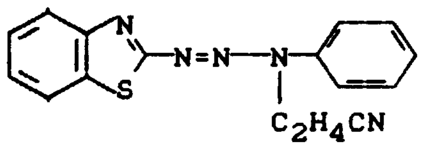 | Acrylamid | 416 |

## Patentansprüche

1. Verfahren zur Herstellung von Farbstoffen der allgemeinen Formel (I)

   D-N = N-Z (I)

   worin

   D für den Rast einer aromatischen oder heterocyclischen Diazokomponente und

   Z für den Rest einer Arylaminogruppe oder den Rest einer Heterocyclyliminogruppe dar Formel

   (II) -N = Het (II)

   steht, worin

   Het für ein heterocyclisches Diradikal steht,

   dadurch gekennzeichnet, daβ eine aromatische oder heterocyclische Diazokomponente der Formel (III)

   $D\text{-}NH_2$ (III)

   und eine Verbindung der Formel (IV)

   $H\text{-}Z^1$ (IV)

   worin

   $Z^1$ für Arylamino oder Hetarylamino steht,

   in wäßrigem Medium in Gegenwart einer salpetrige Säure abgabenden Substanz und in Gegenwart von $CO_2$ bei einem Druck von 5 bis 100 bar und einer Temperatur zwischen 0 und 125 °C umgesetzt werden.

2. Verfahren gemäß Anspruch 1, worin

   D für den Rest einer substituierten oder unsubstituierten aromatischen Diazokomponente mit 6

bis 10 Kohlenstoffatomen oder für den Rast einer substituierten oder unsubstituierten 5-oder 6-gliedrigen heterocyclischen Diazokomponente, die 1 bis 3 Heteroatome aus der Reihe Sauerstoff, Schwefel, Stickstoff enthält und gegebenenfalls einen oder zwei anellierte 5- und/oder 6-gliedrige substituierte oder unsubstituierte carbocyclische Ringe enthält, steht,

Z für den Rest einer substituierten oder unsubstituierten $C_6$-$C_{10}$-Arylaminogruppe oder für den Rest einer substituierten oder unsubstituierten Heterocycyliminogruppe der Formel (II)

-N = Het (II)

steht, worin

Het für das Diradikal eines substituierten oder unsubstituierten gesättigten oder partiell ungesättigten 5- oder 6-gliedrigen heterocyclischen Rings, der 1 bis 3 Heteroatome aus dar Reihe Sauerstoff, Schwefel, Stickstoff enthält und gegebenenfalls durch ein oder zwei 5- und/oder 6-gliedrige substituierte oder unsubstituierte carbocyclische Ringe anelliert ist, steht, und

$Z^1$ für den Rest einer substituierten oder unsubstituierten $C_6$-$C_{10}$-Arylaminogruppe oder für den Rest einer substituierten oder unsubstituierten 5- oder 6-gliedrigen Hetarylaminogruppe die 1 bis 3 Heteroatome aus der Reihe Sauerstoff, Schwefel, Stickstoff im Hetarylteil enthält und gegebenenfalls durch einen oder zwei 5- und/oder 6-gliedrige substituierte oder unsubstituierte carbocyclische Ringe anelliert ist, steht

und wobei als Substituenten für die substituierten aromatischen Diazokomponenten, die substituierten 5- oder 6-gliedrigen heterocyclischen Diazokomponenten, den Arylteil der substituierten $C_6$-$C_{10}$-Arylaminogruppen in der Definition von Z und $Z^1$, den Heterocyclylteil der substituierten Heterocyclyliminogruppen der Formel (II), den Hetarylteil der substituierten Hetarylaminogruppen und die substituierten carbocyclischen Ringe in der Definition von D, Hat und $Z^1$ in Frage kommen: $C_1$-$C_8$-Alkyl, $C_6$-$C_{10}$-Aryl, $C_6$-$C_{10}$-Aryloxy, $C_6$-$C_{10}$-Aryl-$C_1$-$C_4$-alkyl, $C_6$-$C_{10}$-Aryl-$C_1$-$C_4$-alkyloxy, $C_2$-$C_4$-Alkenyl, $C_1$-$C_4$-Alkoxy, $C_1$-$C_4$-Alkoxycarbonyl, $C_1$-$C_4$-Alkylcarbonyloxy, Cyano, $C_1$-$C_4$-(Di)alkylamino, $C_1$-$C_4$-(Di)-alkylaminocarbonyl, $C_1$-$C_4$-Alkylcarbonylamino, Hydroxy, Halogen, $C_1$-$C_4$-Alkylcarbonyl, $C_1$-$C_4$-Alkylsulfonyl, $C_1$-$C_4$-(Di)alkylaminosulfonyl, $C_1$-$C_4$-Alkylsulfonylamino, $C_1$-$C_4$-Alkoxysulfonyl, $C_1$-$C_4$-Alkylsulfonyloxy, Nitro, $C_6$-$C_{10}$-(Di)arylamino, $C_1$-$C_4$-Alkyl-$C_6$-$C_{10}$-arylamino, Phenylsulfonyl, Phenylazo, Banzothiazolyl, 1,2,4-Oxadiazolyl, Morpholino, Piperidino, Piperazino, Pyrrolidino, $C_1$-$C_4$-Alkylmercapto und $C_6$-$C_{10}$-Arylmercapto, und wobei für den Aminoteil der substituierten $C_6$-$C_{10}$-Arylaminogruppen in der Definition von Z und $Z^1$ als Substituenten gegebenenfalls substituiertes $C_1$-$C_8$-Alkyl, $C_2$-$C_4$-Alkenyl, substituiertes oder unsubstituiertes $C_1$-$C_4$-Alkyl-$C_6$-$C_{10}$-aryl in Frage kommen.

**3.** Verfahren gemäß Ansprüchen 1 und 2
worin

D für den Rest einer substituierten oder unsubstituierten aromatischen 6 bis 10 Kohlenstoffatome enthaltenden Diazokomponente oder für den Rest einer heterocyclischen Diazokomponente aus der Reihe der jeweils gegebenenfalls substituierten und jeweils gegebenenfalls benzanellierten Thiazole, Isothiazole, Thiadiazole, Oxazole, Imidazole und Triazole steht, und

Z für einen Rest der Formel

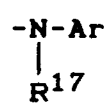

(V)

oder -N = Het (II)

steht, worin

Ar für jeweils gegebenenfalls substituiertes Phenyl oder Naphthyl steht,

$R^{17}$ für Wasserstoff, gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiertes $C_1$-$C_8$-Alkyl steht oder für $C_2$-$C_4$-Alkylen, für jeweils gegebenenfalls durch Halogen, Cyano, $C_1$-$C_4$-Alkyl oder $C_1$-$C_4$-Alkoxy substituiertes Phenylmethyl oder Phenylethyl steht,

Het für das Diradikal eines partiell ungesättigten, 5-gliedrigen, 1 bis 3 Heterostome aus der

Reihe Sauerstoff, Schwefel, Stickstoff enthaltenden heterocyclischen Rings, der gegebenenfalls durch einen substituierten oder unsubstituierten Benzolring anelliert ist steht, wobei der heterocyclische Ring gegebenenfalls 1-bis 3-fach gleich oder verschieden substituiert ist durch $C_1$-$C_8$-Alkyl, das gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiert ist, weiterhin durch $C_2$-$C_4$-Alkenyl, oder durch Phenylmethyl oder Phenylethyl, welches jeweils gegebenenfalls durch Halogen, Cyano, $C_1$-$C_4$-Alkyl, $C_1$-$C_4$-Alkoxy, $C_1$-$C_4$-(Di)alkylamino, $C_6$-$C_{10}$-(Di)arylamino, $C_1$-$C_4$-Alkyl-$C_6$-$C_{10}$-arylamino, Morpholino, Piperidino, Piperazino, Pyrrolidino, $C_1$-$C_4$-Alkylmercapto oder $C_6$-$C_{10}$-Arylmercapto substituiert ist,
und

$Z^1$ für einen Rest der Formel

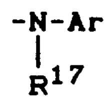 (V)

oder

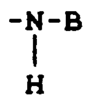 (VI)

steht, worin
Ar und $R^{17}$ die oben angegebene Bedeutung haben, und

B für den Rest eines 5-gliedrigen ungesättigten heterocyclischen Rings steht, der 1 bis 3 Heteroatome aus der Reihe Sauerstoff, Schwefel, Stickstoff enthält und der gegebenenfalls durch einen substituierten oder unsubstituierten Benzolring anelliert ist, wobei der heterocyclische Ring gegebenenfalls 1- bis 3-fach gleich oder verschieden substituiert ist durch $C_1$-$C_8$-Alkyl, welches gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiert ist, durch $C_2$-$C_4$-Alkenyl oder durch Phenylmethyl oder Phenylethyl, welches jeweils gegebenenfalls durch Halogen, Cyano, $C_1$-$C_4$-Alkyl oder $C_1$-$C_4$-Alkoxy, $C_1$-$C_4$- (Di)alkylamino, $C_6$-$C_{10}$-(Di)arylamino, $C_1$-$C_4$-Alkyl-$C_6$-$C_{10}$-arylamino, Morpholino, Piparidino, Piperazino, Pyrrolidino, $C_1$-$C_4$-Alkylmercapto oder $C_6$-$C_{10}$-Arylmercapto substituiert ist,

und wobei als Substituenten für die substituierten aromatischen Diazokomponenten und die substituierten Phenyl- und Naphthyl-Reste in der Definition von Z und $Z^1$ und die anellierten Benzolringe in der Definition von Het und B in Frage kommen:
$C_1$-$C_8$-Alkyl, $C_6$-$C_{10}$-Aryl, $C_6$-$C_{10}$-Aryloxy, $C_6$-$C_{10}$-Aryl-$C_1$-$C_4$-alkyl-, $C_6$-$C_{10}$-Aryl-$C_1$-$C_4$-alkyloxy, $C_2$-$C_4$-Alkenyl, $C_1$-$C_4$-Alkoxy, $C_1$-$C_4$-Alkoxycarbonyl, $C_1$-$C_4$-Alkylcarbonyloxy,-Cyano, $C_1$-$C_4$-(Di)alkylamino, $C_1$-$C_4$-(Di)alkylaminocarbonyl, $C_1$-$C_4$-Alkylcarbonylamino, Hydroxy, Halogen, $C_1$-$C_4$-Alkylcarbonyl, $C_1$-$C_4$-Alkylsulfonyl, $C_1$-$C_4$-(Di)alkylaminosulfonyl, $C_1$-$C_4$-Alkylsulfonylamino, $C_1$-$C_4$-Alkoxysulfonyl, $C_1$-$C_4$-Alkylsulfonyloxy, Nitro, $C_6$-$C_{10}$-(Di)arylamino, $C_1$-$C_4$-Alkyl-$C_6$-$C_{10}$- arylamino, Phenylsulfonyl, Phenylazo, Benzothiazolyl, 1,2,4-Oxadiazolyl, Morpholino, Piparidino, Piparazino, Pyrrolidino, $C_1$-$C_4$- Alkylmercapto und $C_6$-$C_{10}$-Arylmercapto, und wobei für den Aminoteil der substituierten $C_6$-$C_{10}$-Arylaminogruppen in der Definition von Z und $Z^1$ als Substituenten gegebenenfalls substituiertes $C_1$-$C_8$-Alkyl, $C_2$-$C_4$-Alkenyl, substituiertes oder unsubstituiertes $C_1$-$C_4$-Alkyl-$C_6$-$C_{10}$-aryl in Frage kommen.

**4.** Verfahren gemäß Ansprüchen 1 bis 3, worin
D für einen Rest der Formel

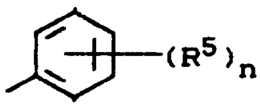 , 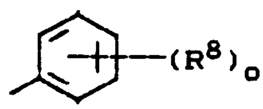 ,

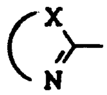

oder

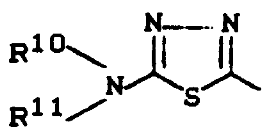

steht, worin

$R^5$ für Wasserstoff, Halogen $C_1$-$C_4$-Alkyl, $C_1$-$C_4$-Alkoxy, Benzyl, Benzyloxy, für jeweils gegebenenfalls durch Halogen, $C_1$-$C_4$-Alkyl, $C_1$-$C_4$-Alkoxy, Acetyl, Benzoyl oder $C_1$-$C_4$-Alkoxycarbonyl substituiertes Phenyl oder Phenyloxy oder für Phenylazo steht, oder

$R^5$ zusammen mit dem Benzolring an den es gebunden ist für ein Tetralin-, Naphthalin- oder Benzodioxan-System steht,

n für 1 oder 2 steht,

$R^8$ für Wasserstoff, Halogen, $C_1$-$C_4$-Alkyl, $C_1$-$C_4$-Alkoxy, $C_1$-$C_4$-(Di)alkylamino, $C_1$-$C_4$-(Tri)alkylammonium, Benzyl, Benzyloxy, Phenyloxy, Acetyl, Benzoyl, $C_1$-$C_4$-Alkoxycarbonyl, Carboxamido, Carboxyl, Nitro, Sulfonamido oder Sulfo steht,

o für 1 oder 2 steht,

X für die restlichen Glieder eines Thiazol-, Isothiazol-, Benzthiazol-, 1,2,4-Triazol- oder 1,3,4-Thiadiazolrings steht,

$R^{10}$ und $R^{11}$ unabhängig voneinander für Wasserstoff oder für gegebenenfalls durch Halogen, OH, Cyano, $C_1$-$C_4$-Alkoxy, $C_1$-$C_4$-Carbonyloxy, Phenoxy oder Phenyl substituiertes $C_1$-$C_4$-Alkyl stehen,

oder

$R^{10}$ und $R^{11}$ zusammen mit dem Stickstoffatom an das sie gebunden sind für die restlichen Glieder eines 5- oder 6-gliedrigen hetcrocyclischen Rings, der ein weiteres Heteroatom aus der Reihe Sauerstoff, Schwefel, Stickstoff enthalten kann, stehen,

$R^{10}$ weiterhin gegebenenfalls durch Halogen, $C_1$-$C_4$-Alkyl oder $C_1$-$C_4$-Alkoxy substituiertes Phenyl steht,

und

$R^{11}$ weiterhin für einen Alkylenrest steht, der durch Ringschluß mit dem Thiadiazolring einen partiell ungesättigten 5- oder 6-gliedrigen heterocyclischen Ring bildet,

Z für den Rest der Formel

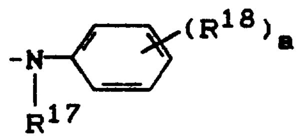 ,

(VII)

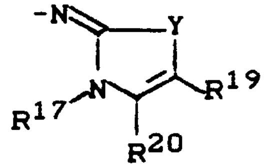 ,

(VIII)

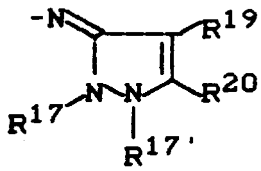 ,

(IX)

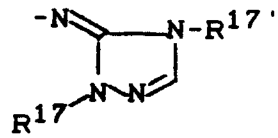 ,

(X)

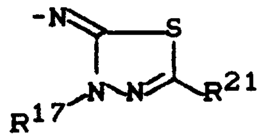

(XI)

oder

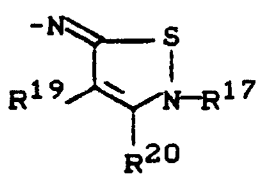

(XII)

steht, worin

Y für -S-, -O- oder

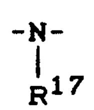

steht,

worin

$R^{17}$ und $R^{17'}$ unabhängig voneinander für Wasserstoff, gegebenenfalls durch Hydroxy, Halogen, Cyano, $C_1$-$C_4$-Alkoxy, Carboxyl, $C_1$-$C_4$-(Di)alkylaminocarbonyl, $C_1$-$C_4$-Alkoxycarbonyl oder $C_1$-$C_4$-Alkylsulfonyl substituiertes $C_1$-$C_8$-Alkyl steht, oder für $C_2$-$C_4$-Alkenyl, oder für jeweils gegebenenfalls durch Halogen, Cyano, $C_1$-$C_4$-Alkyl oder $C_1$-$C_4$-Alkoxy substituiertes Phenylmethyl oder Phenylethyl stehen,

$R^{18}$ für $C_1$-$C_4$-Alkyl, $C_1$-$C_4$-Alkoxy, Halogen, Nitro, Cyano, $C_1$-$C_4$-Alkylsulfonyl, Phenylsulfonyl, $C_1$-$C_4$-Alkylcarbonylamino, Phenylazo, Benzothiazolyl oder 1,2,4-Oxadiazolyl steht,

a für 0, 1, 2 oder 3 steht,

$R^{19}$ und $R^{20}$ entweder für Wasserstoff stehen oder
gemeinsam für die restlichen Glieder eines anellierten Benzolrings stehen und

$R^{21}$ für $C_1$-$C_4$-(Di)alkylamino, $C_6$-$C_{10}$-(Di)arylamino, $C_1$-$C_4$-Alkyl-$C_6$-$C_{10}$-arylamino, Morpholino, Piperidino, Piperazino, Pyrrolidino, $C_1$-$C_4$-Alkylmercapto oder $C_6$-$C_{10}$-Arylmercapto steht,

und

$Z^1$ für einen Rest dar Formel

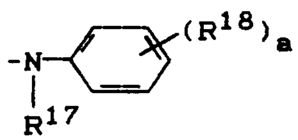

,

(XIII)

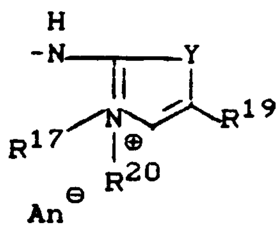

,

(XIV)

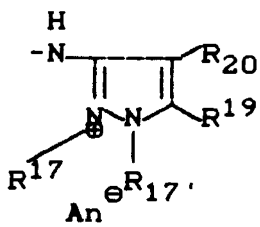

,

(XV)

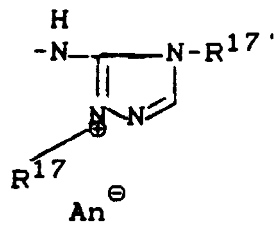

,

(XVI)

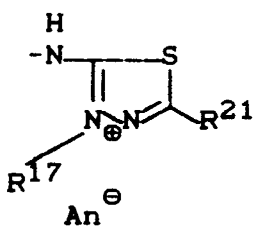

(XVII)

oder

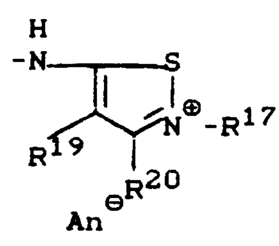

(XVIII)

steht, worin
Y, $R^{17}$, $R^{17'}$, $R^{18}$, $R^{19}$, $R^{20}$, $R^{21}$ und a die oben angegebene Bedeutung haben und

An$^{\ominus}$ für ein Anion aus der Reihe Cl$^{\ominus}$` $CH_3OSO_2O^{\ominus}$, I$^{\ominus}$, Br$^{\ominus}$, $CH_3COO^{\ominus}$ steht.

**5.** Verfahren gemäß Ansprüchen 1 bis 4 zur Herstellung von Farbstoffen der Formel

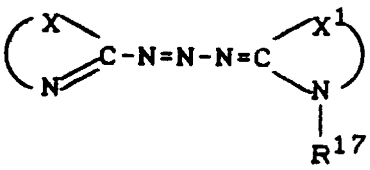

(XIX)

worin

X für die restlichen Glieder eines Thiazol-, Isothiazol-, Benzthiazol-, 1,2,4-Triazol- oder 1,3,4-Thiadiazolrings steht,

$X^1$ für die restlichen Glieder eines Thiazolin-, Isothiazolin-, Benzthiazolin-, 1,2,4-Triazolin- oder 1,3,4-Thiadiazolinrings steht,

$R^{17}$ für gegebenenfalls durch Cyano, $C_1$-$C_4$-(Di)alkylaminocarbonyl, Hydroxyl, $C_1$-$C_4$-Alkoxy, Carboxyl, $C_1$-$C_4$-Alkoxycarbonyl oder $C_1$-$C_4$-Alkylsulfonyl substituiertes $C_1$-$C_4$-Alkyl steht,

indem ein heterocyclisches Amin der Formel

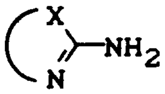

(XX)

worin X die oben angegebene Bedeutung hat,
und eine heterocyclische Ammoniumverbindung der Formel

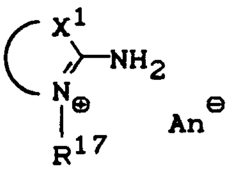

(XXI)

worin

$X^1$ und $R^{17}$ die oben angegebene Bedeutung haben und

An$^{\ominus}$ für ein Anion aus der Reihe Cl$^{\ominus}$, $CH_3OSO_2O^{\ominus}$, I$^{\ominus}$, Br$^{\ominus}$, $CH_3COO^{\ominus}$ steht,

in wäßrigem Medium in Gegenwart einer salpetrige Säure abgebenden Substanz und in Gegenwart von $CO_2$ bei einem Druck von 5 bis 100 bar und einer Temperatur zwischen 0 und 125 °C umgesetzt werden.

**6.** Verfahren gemäß Ansprüchen 1 bis 5, dadurch gekennzeichnet, daß die Umsetzung bei einer Temperatur zwischen 30 und 40 °C erfolgt.

**7.** Verfahren gemäß Ansprüchen 1 bis 6, dadurch gekennzeichnet, daß es sich bei der salpetrigen Säure abgebenden Substanz um ein anorganisches oder organisches Nitrit handelt.

**8.** Verfahren gemäß Ansprüchen 1 bis 7, dadurch gekennzeichnet, daß die Umsetzung während einer Zeit von 10 Minuten bis 24 Stunden erfolgt.

**9.** Verfahren gemäß Ansprüchen 1 bis 8, dadurch gekennzeichnet, daß die Umsetzung in Wasser, gegebenenfalls in Mischung mit organischen Lösungsmitteln die ganz oder teilweise mit Wasser mischbar sind erfolgt.

**10.** Verfahren gemäß Ansprüchen 1 bis 9, dadurch gekennzeichnet, daß die Umsetzung in einem Gemisch aus Wasser und Methanol im Verhältnis 1:2 erfolgt.

Europäisches Patentamt

# EUROPÄISCHER RECHERCHENBERICHT

Nummer der Anmeldung
EP 93 10 6056

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 446 731 (BAYER AG)<br>* Seite 2, Zeile 1 - Zeile 52 *<br>--- | 1-10 | C09B26/06<br>C09B56/20<br>C09B41/00 |
| A | GB-A-2 017 134 (BAYER AG)<br>* Seite 9, Zeile 6 - Zeile 10 *<br>--- | 1-10 | |
| A<br><br><br>D | EP-A-0 053 751 (BAYER AG)<br>* Seite 8, Zeile 3 - Zeile 14 *<br>* Beispiele 1,17,32 *<br>& DE-A-3 045 912<br>--- | 1-10 | |
| D,A | DE-A-1 069 563 (J. R. GEIGY AG)<br>* Spalte 3, Zeile 47 - Zeile 61; Beispiel 2 *<br>--- | 1-10 | |
| A | DE-A-3 035 056 (BAYER AG)<br>* Seite 4, Zeile 15 - Seite 5, Zeile 11 *<br>* Seite 7, Zeile 10 - Seite 8, Zeile 17 *<br>--- | 1-10 | |
| A | PATENT ABSTRACTS OF JAPAN<br>vol. 6, no. 177 (C-124)(1055) 11. September 1982<br>& JP-A-57 091 975 ( DOUJIN KAGAKU KENKYUSHO K. K. ) 8. Juni 1982<br>* Zusammenfassung *<br>----- | 1 | RECHERCHIERTE SACHGEBIETE (Int. Cl.5 )<br>C09B<br>C07D |

Der vorliegende Recherchenbericht wurde für alle Patentansprüche erstellt

| Recherchenort | Abschlußdatum der Recherche | Prüfer |
|---|---|---|
| DEN HAAG | 05 AUGUST 1993 | KETTERER M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)